# FASCICULE DE BREVET EUROPEEN

(11) **EP 1 154 740 B1**
(45) Date de publication et mention de la délivrance du brevet: **01.09.2004**
(21) Numéro de dépôt: 00901180.0
(22) Date de dépôt: 26.01.2000
(51) Int. Cl.: A61F 2/44

(54) **IMPLANT VERTEBRAL INTERSOMATIQUE A INTRODUCTION SAGITTALE**
ZWISCHENKÖRPERLICHES WIRBELIMPLANTAT ZUR SAGITTALEN EINFÜHRUNG
INTERBODY VERTEBRAL IMPLANT WITH SAGITTAL INSERTION

(30) Priorité: 26.01.1999 FR 9900982
(43) Date de publication de la demande: 21.11.2001
(73) Titulaire: SCIENT'X, 75007 Paris (FR)
(72) Inventeur: COMMARMOND, Jacques, F-03200 Vichy (FR)
(74) Mandataire: Thibault, Jean-Marc
(86) Numéro de dépôt international: PCT/FR2000/000176
(87) Numéro de publication internationale: WO 2000/044318

(56) Documents cités:
- EP-A- 0 493 698
- EP-A- 0 599 419
- EP-A- 0 834 295
- WO-A-96/14809
- WO-A-97/23174
- WO-A-98/17209
- FR-A- 2 724 312

## Description

### DOMAINE TECHNIQUE :

La présente invention concerne un implant intersomatique destiné à être inséré dans l'espace discal défini entre deux vertèbres adjacentes en vue de restituer une hauteur convenable intervertébrale et d'assurer une fusion osseuse entre lesdites vertèbres adjacentes.

L'objet de l'invention est plus particulièrement destiné au rétablissement de l'espace discal de la partie lombaire de la colonne vertébrale d'un patient.

### TECHNIQUE ANTERIEURE :

Dans l'état de la technique, il est connu d'insérer un implant intersomatique dans l'espace discal défini entre deux vertèbres adjacentes. De nombreuses formes de réalisation de tels implants intersomatiques ont été proposées dans l'art antérieur. Par exemple, il est connu par le brevet **FR 2 724 312,** un implant intersomatique lombaire se présentant sous la forme d'une cage comportant deux parois sagittales reliées entre elles par une paroi transversale antérieure et une paroi transversale postérieure. Les parois délimitent entre elles un volume ouvert destiné à recevoir un matériel osseux spongieux permettant de favoriser la fusion osseuse entre les deux vertèbres. Un tel implant présente une partie d'extrémité élargie lui conférant une configuration réniforme permettant d'obtenir une surface d'appui relativement importante. De plus, les rebords inférieur et supérieur des parois de la cage sont munis de crans pour assurer sa retenue, par ancrage dans l'os de chaque vertèbre adjacente. Un tel implant après son introduction selon une direction sagittale, est conçue pour éviter sa migration aussi bien latérale que longitudinale.

Si un tel implant présente une surface d'appui importante grâce à sa forme réniforme, il présente l'inconvénient de nécessiter parfois une voie d'abord relativement large. Il est connu, par ailleurs, notamment par le document **EP 0 493 698**, une cage de forme générale parallélèpipédique, offrant l'avantage de limiter la voie d'abord. Il apparaît, cependant difficile, dans certaines conditions, de placer l'implant dans l'espace intervertébral dans une position telle qu'il puisse assurer une stabilité optimum entre les vertèbres adjacentes, alors que cette stabilité est indispensable pour obtenir une bonne fusion osseuse.

Le document WO-98 17 209 montre les caractéristiques du preambule de la revendication 1 (figures 40-42).

### EXPOSE DE L'INVENTION :

La Déposante a eu le mérite de concevoir un implant intersomatique lombaire adapté pour offrir la possibilité de le placer dans une position telle qu'il puisse permettre d'obtenir une stabilité accrue entre deux vertèbres adjacentes en augmentant le polygone de sustentation tout en limitant, bien entendu, la voie d'abord.

L'objet de l'invention vise donc à satisfaire à ce besoin en proposant un implant intersomatique lombaire destiné à être inséré entre deux vertèbres adjacentes par une voie d'abord limitée tout en permettant son positionnement au voisinage des bords latéraux des vertèbres en vue d'augmenter le polygone de sustentation et, par suite, la stabilité entre lesdites vertèbres adjacentes.

Pour atteindre un tel objectif, l'implant selon l'invention est destiné à être inséré dans l'espace discal défini entre deux vertèbres adjacentes en vue du rétablissement anatomique de l'espace intervertébral, l'implant se présentant sous la forme d'une cage de forme générale parallélèpipédique comportant deux parois sagittales reliées entre elles par au moins une paroi transversale antérieure et une paroi transversale postérieure, les parois délimitant entre elles un volume ouvert pour un remplissage osseux.

Selon l'invention, l'une des parois sagittales, dite externe de l'implant, présente :
- un rebord supérieur et un rebord inférieur aménagés chacun pour présenter au moins une arête de rétention s'étendant sensiblement dans le plan sagittal, autorisant une introduction par une voie postérieure,
- et une face externe aménagée pour présenter une conformation d'aide à la pénétration permettant un décalage latéral de la cage jusqu'à une position de blocage dans l'espace discal, obtenue par l'arête de rétention empêchant le recul latéral de la cage.

Conformément à l'invention, l'implant intersomatique est introduit dans l'espace intervertébral selon une direction sensiblement parallèle au plan sagittal et se trouve conçu pour pouvoir être déplacé latéralement, afin de venir à proximité des bords latéraux des vertèbres pour permettre d'augmenter le polygone de sustentation entre lesdites deux vertèbres.

L'objet de l'invention trouve une application particulièrement avantageuse pour être mis en place grâce à une voie unilatérale postérieure plus conservatrice pour le patient par rapport à une large voie classique d'abord. En effet, il peut être envisagé de placer un ou deux implants intersomatiques lombaires à partir d'une voie postérieure unilatérale tout en obtenant une stabilité accrue entre lesdites vertèbres adjacentes. La mise en place d'un tel implant par une voie postérieure unilatérale permet d'éviter d'interrompre la structure des épineuses, de forcer durement et longuement les muscles paravertébraux et d'infliger une fibrose circonférentielle.

Diverses autres caractéristiques ressortent de la description faite ci-dessous en référence aux dessins annexés qui montrent, à titre d'exemples non limitatifs, des formes de réalisation et de mise en oeuvre de l'objet de l'invention.

### BREVE DESCRIPTION DES DESSINS :

La **fig. 1** est une vue en perspective ¾ avant d'un exemple de réalisation d'un implant conforme à l'invention.
La **fig. 2** est une en perspective opposée ¾ arrière de l'implant illustré à la **fig. 1.**
La **fig. 3** est une vue de dessus d'un implant conforme à l'invention.
La **fig. 4** est une vue de profil avant d'un implant conforme à l'invention.
La **fig. 5** est une vue de profil arrière d'un implant conforme à l'invention.
La **fig. 6** est une vue de profil latéral dit inférieur, en considération de la vue de dessus de la **fig. 3.**
La **fig. 7** est une vue de profil latéral dit supérieur, en considération de la vue de dessus de la **fig. 3.**
La **fig. 8** est une vue de profil montrant un détail caractéristique d'une variante de réalisation de l'invention.
La **fig. 9** est une vue de dessus d'un exemple de mise en place d'implants intersomatiques lombaires dans un espace discal.

### MEILLEURE MANIERE DE REALISER L'INVENTION :

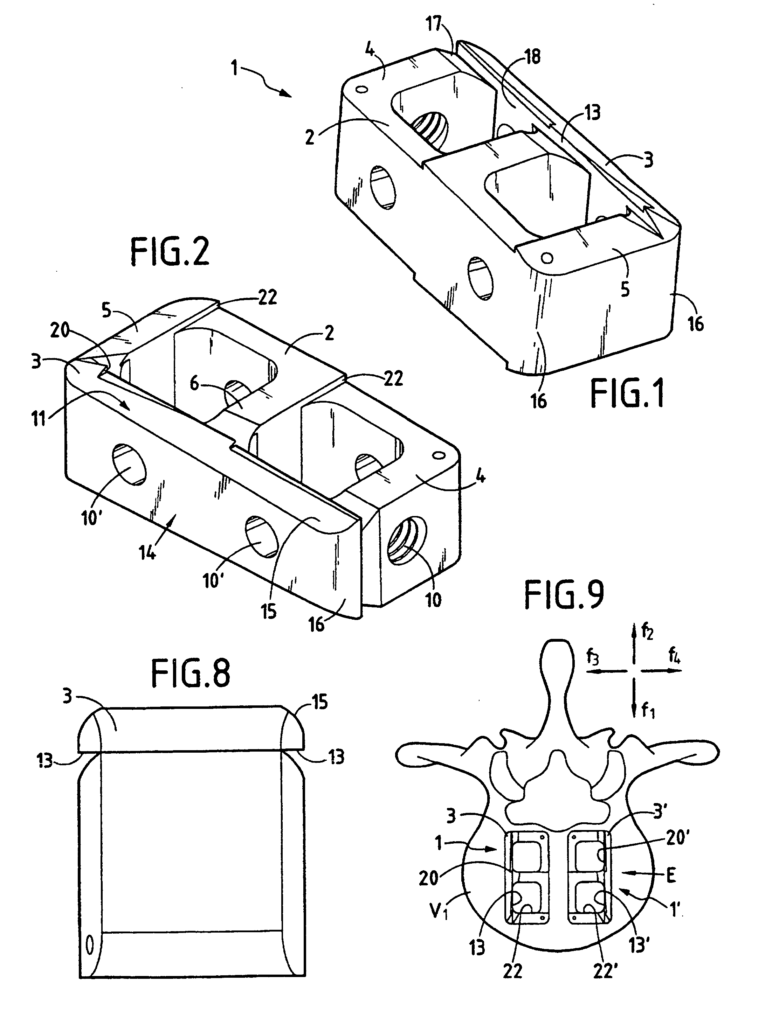

Tel que cela apparaît plus précisément aux **fig. 1** à **7,** l'implant intersomatique lombaire conforme à l'invention se présente sous la forme d'une cage **1** présentant une forme générale parallélèpipédique et destinée à être insérée dans l'espace discal entre deux vertèbres adjacentes. La cage **1** comporte une première paroi sagittale **2** dite interne, et une seconde paroi sagittale **3**, dite externe, s'étendant sensiblement parallèlement l'une à l'autre et à un plan **S**, dit sagittal ou antéro-postérieur. Les parois sagittales **2** et **3** sont reliées entre elles par une paroi transversale, dite postérieure **4**, et par une paroi transversale antérieure **5** s'étendant parallèlement l'une à l'autre et à un plan vertical transversal **T** perpendiculaire au plan sagittal **S**.

Dans un exemple préféré de réalisation illustré sur les dessins, la cage **1** comporte au moins une paroi transversale intermédiaire **6** reliée aux parois sagittales **2** et **3** et s'étendant parallèlement aux parois transversales **4** et **5** et à distance de ces dernières. Dans l'exemple illustré, la paroi transversale intermédiaire **6** constitue une paroi médiane par rapport aux parois transversales **4** et **5**. La cage **1** présente ainsi intérieurement deux volumes **7** s'ouvrant sur les faces supérieure **8** et inférieure **9** de la cage. Les volumes **7** sont destinés à être remplis par de l'os spongieux destiné à la fusion intersomatique. De manière classique, la paroi transversale postérieure **4** comporte un trou **10** éventuellement fileté débouchant à l'intérieur du volume **7.** Le trou **10** est destiné au montage d'un outil de préhension et de positionnement de la cage entre les vertèbres et permet, ensuite, de favoriser la fusion osseuse. Selon une caractéristique préférée de réalisation, chaque paroi sagittale **2, 3** est pourvue de deux trous de passage **10'** communiquant chacun avec un volume **7**.

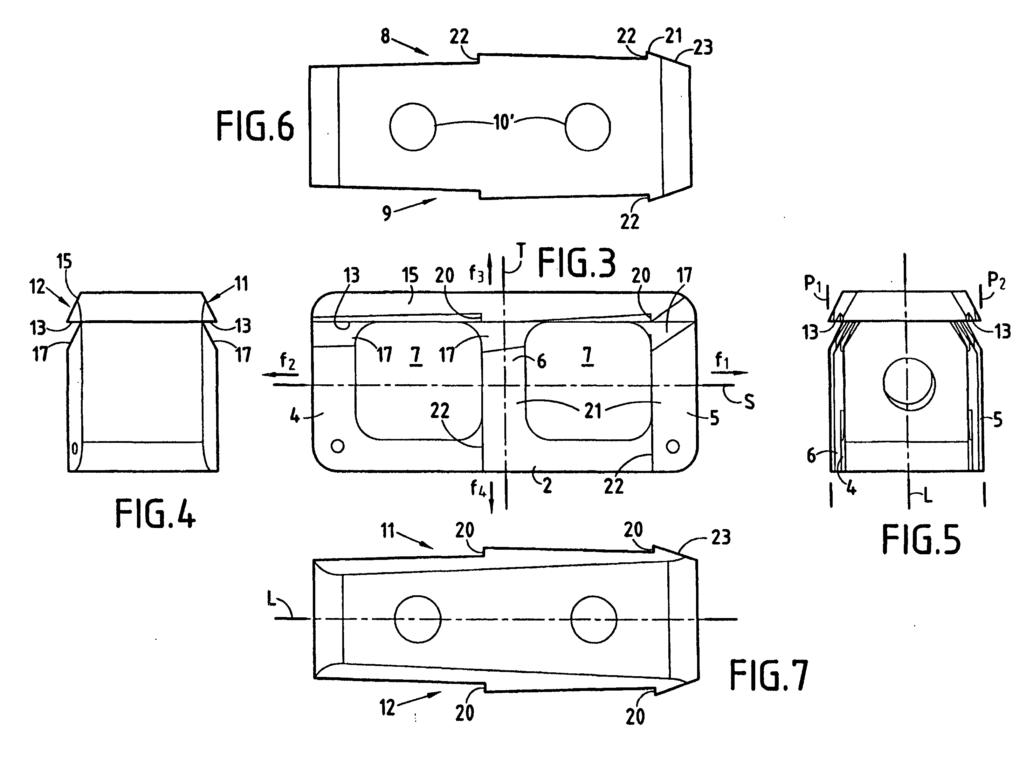

La paroi sagittale, dite externe **3**, comporte un rebord supérieur **11** et un rebord inférieur **12** s'étendant respectivement selon les faces supérieure **8** et inférieure **9**. Conformément à l'invention, chaque rebord **11**, **12** est aménagé pour présenter au moins une arête de rétention **13** s'étendant dans un plan parallèle au plan sagittal **S,** comme le montre clairement la **fig. 3.** Compte tenu de l'extension de l'arête de rétention **13** dans le plan sagittal **S,** la cage peut être déplacée dans les deux sens d'une direction dite sagittale représentée par les flèches **f**_{**1**} et **f**_{**2**} et qui est perpendiculaire au plan transversal **T**. Dans l'exemple illustré, le sens d'introduction **f**_{**1**} opposé au sens **f**_{**2**} est celui pour lequel la paroi transversale antérieure **5** est située en aval par rapport à la paroi transversale postérieure **4.**

Selon une autre caractéristique de l'invention, la paroi sagittale externe **3** présente une face externe **14** aménagée pour présenter une conformation **15** d'aide à la pénétration permettant un décalage latéral de la cage dans un sens **f**_{**3**} selon une direction perpendiculaire au plan sagittal **S** et pour lequel la paroi externe **3** est en aval par rapport à la paroi interne **2.** Cette conformation d'aide à la pénétration **15** autorise un déplacement latéral de la cage selon le sens **f**_{**3**} jusqu'à une position de blocage obtenue par l'arête de rétention **13** empêchant le recul latéral de la cage selon un sens **f**_{**4**} opposé au sens **f**_{**3**}. Tel que cela ressort plus précisément des **fig. 4** et **5,** chaque arête de rétention **13** réalisée sur la paroi sagittale externe **3** est prolongée transversalement vers l'extérieur, par une conformation **15** d'aide à la pénétration constituée, dans l'exemple illustré, par un chanfrein, de sorte que ladite paroi sagittale **3** présente, selon le plan transversal **T,** un profil tronconique dont la petite base est formée par la face externe de la paroi sagittale **3.** Bien entendu, il pourrait être envisagé de conférer à la face externe **14** de la paroi sagittale, un profil différent tout en permettant l'introduction de la cage dans le sens **f**_{**3**}. Par exemple, il pourrait être envisagé de remplacer chaque chanfrein par un profil arrondi **15,** comme illustré à la **fig. 8**. Dans le même sens, et tel que cela ressort plus précisément des **fig. 1** et **2,** la face externe **14** de la paroi sagittale externe **3** est raccordée aux parois transversales **4, 5** par des congés **16.** De même, la paroi sagittale interne **2** est raccordée aux parois transversales **4**, **5** par des congés **16** permettant de disposer d'une cage avec des coins arrondis.

De préférence, chaque arête de rétention **13** est obtenue par un biseautage réalisé dans les parties de raccordement **17** des parois transversales **4, 5, 6** avec la paroi sagittale externe **3.** Ainsi, chaque arête de rétention **13** est constituée par la partie longitudinale supérieure de la face interne **18** de la paroi sagittale externe **3** formant ainsi une dent, un cran ou une bordure saillante par rapport aux parois transversales **4**, **5**, **6**. Dans l'exemple illustré sur les dessins, l'arête de rétention **13** s'étend de manière continue sur toute la longueur de la cage, de la paroi postérieure **4** jusqu'à la paroi antérieure **5.** Bien entendu, il peut être envisagé que l'arête de rétention **13** s'étende sur une partie seulement de la longueur de la cage suivant un ou plusieurs tronçons. Dans le même sens, la paroi sagittale externe **3** peut être aménagée pour présenter plusieurs arêtes de rétention **13** décalées entre elles en s'étendant chacune dans un plan parallèle au plan sagittal **S.** De plus, chaque arête de rétention **13** peut présenter dans le plan sagittal **S,** un profil rectiligne ou convexe.

Selon une variante préférée de réalisation, la cage **1** présente une symétrie par rapport à un plan longitudinal médian **L** perpendiculaire au plan sagittal **S** et au plan transversal **T**. Ainsi, une cage **1** après retournement selon un axe longitudinal, peut occuper une position symétrique avec une autre cage analogue.

Selon une première forme de réalisation, chaque arête de rétention **13** s'étend sensiblement dans les plans d'extension extrêmes supérieur **P**_{**1**} et inférieur **P**_{**2**} des parois de la cage. Ainsi, tel que cela apparaît sur la **fig. 5,** les arêtes de rétention **13** ne dépassent pas les plans supérieur **P**_{**1**} et inférieur **P**_{**2**}, parallèles au plan longitudinal **L** et dans lesquels s'étendent les rebords extrêmes des parois **2, 4, 5** ou **6.** Il est à noter qu'il peut être envisagé que chaque arête de rétention **13** s'étende en saillie par rapport aux plans d'extension extrêmes inférieur **P**_{**1**} et supérieur **P**_{**2**} des parois **2, 4, 5** ou **6,** afin de conférer à la cage un effet distractant lors de son introduction dans l'espace intervertébral. Dans cette dernière forme de réalisation, chaque arête de rétention **13** s'étend au-delà des rebords des parois transversales **4, 5, 6** et de la paroi sagittale interne **2.**

Selon une autre caractéristique de l'invention, chaque arête de rétention **13** est taillée localement pour délimiter au moins un, et dans l'exemple illustré, deux redents **20** constituant une butée s'opposant au recul de la cage dans le plan sagittal selon le sens **f**_{**2**} (**fig. 3** et **7**). Dans le même sens, au moins l'une et, dans l'exemple illustré, les parois transversales antérieure **5** et intermédiaire **6** présentent des rebords inférieur et supérieur **21** aménagés chacun pour présenter au moins une butée ou une dent de rétention **22** empêchant le recul de la cage selon le sens **f**_{**2**} (**fig. 3** et **6**). Dans l'exemple illustré, chaque dent de rétention **22** s'étend dans un plan transversal commun à celui d'extension d'un redent **20.** Tel que cela ressort plus précisément des **fig. 2, 3** et **6,** chaque dent de rétention **22** est constituée par la partie supérieure de la face interne des parois transversales antérieure **5** et intermédiaire **6**. Ainsi, les rebords des parois sagittales **2, 3,** prises entre les faces internes des parois transversales antérieure **5** et intermédiaire **6**, ainsi que le rebord de la paroi transversale intermédiaire **6,** sont taillées obliquement pour faire apparaître les dents de rétention **22** et les redents **20,** de sorte que la cage présente sur cette partie de sa longueur, un profil tronconique dans le plan sagittal S (**fig. 6**). De même, le rebord de la paroi transversale postérieure **4,** ainsi que les rebords des parois sagittales **2, 3** prises entre la face interne de la paroi transversale intermédiaire **6** jusqu'à la face externe de la paroi transversale postérieure **4,** sont taillés selon un plan sensiblement parallèle au plan longitudinal **L**, de sorte que la cage présente sur cette partie de sa longueur, un profil sensiblement rectangulaire dans le plan sagittal **S.** Par ailleurs, chaque rebord **23** de la paroi transversale antérieure **5** est taillé obliquement, de sorte que le profil de cette paroi dans le plan sagittal, est tronconique dont la plus petite base est formée par la face externe de la paroi **5**.

Selon une variante préférée de réalisation et tel que cela apparaît plus précisément aux **fig. 5** et **7**, les parois transversales **4, 5, 6** présentent dans le plan sagittal **S** des hauteurs déterminées différentes définissant une pente adaptée à la lordose physiologique de l'espace intervertébral.

Tel que cela ressort de la **fig. 9,** la cage **1** conforme à l'invention est destinée à être insérée dans l'espace discal **E** défini entre deux vertèbres adjacentes, dont une **V**_{**1**} à été représentée. La cage **1** présente la particularité de pouvoir être introduite dans l'espace discal selon une direction sensiblement parallèle à son plan sagittal dans le sens **f**_{**1**} et à être décalé latéralement selon le sens **f**_{**3**} jusqu'à une position de blocage dans l'espace discal obtenue par les arêtes de rétention **13** venant s'ancrer dans les plateaux des vertèbres, empêchant le recul de la cage selon un sens opposé **f**_{**4**}. De même, le blocage de la cage **1** dans le plan sagittal **S** selon le sens **f**_{**2**} est assuré par les redents **20** et les dents **22,** destinés à coopérer avec les plateaux des vertèbres.

### POSSIBILITE D'APPLICATION INDUSTRIELLE :

L'objet de l'invention est particulièrement adapté pour permettre une arthrodèse lombaire intersomatique avec deux cages **1** introduites par une voie postérieure unilatérale. Selon cette technique, il est prévu de réaliser sur le patient, une voie d'abord unilatérale postérieure, à partir de laquelle est introduite une première cage **1** dans l'espace discal par un déplacement sensiblement selon son plan sagittal **S** suivi d'un décalage latéral. Un tel implant peut donc être placé dans l'espace discal à l'opposé de la voie d'abord. Une deuxième cage **1'** peut alors être introduite par la même voie d'abord, en étant orientée pour être symétrique par rapport à la première cage **1.** Cette cage **1'** est identique à la cage 1 mais est simplement retournée avant son introduction dans l'espace discal. Tel que cela apparaît clairement à la **fig. 9,** les cages **1, 1'** sont positionnées de manière que les parois sagittales externes **3, 3'** se trouvent placées du côté extérieur des plateaux des vertèbres. Compte tenu du positionnement symétrique de la cage **1'** par rapport à la cage **1,** la cage **1'** est bloquée latéralement dans le sens **f**_{**3**} par les arêtes de rétention **13'**, tandis que le blocage de la cage **1'** dans le plan sagittal **S** selon le sens **f**_{**1**} est assuré par les redents **20'** et les dents **22'.**

## Revendications

1. Implant intersomatique lombaire destiné à être inséré dans l'espace discal défini entre deux vertèbres adjacentes en vue du rétablissement anatomique de l'espace intervertébral, l'implant se présentant sous la forme d'une cage (**1**) de forme générale parallélèpipédique comportant deux parois sagittales (**2, 3**) reliées entre elles par au moins une paroi transversale antérieure (**5**) et une paroi transversale postérieure (**4**), les parois délimitant entre elles un volume ouvert (**7**) pour un remplissage osseux,
l'une des parois sagittales, dite externe (**3**) de l'implant, présentant:
- un rebord supérieur (**11**) et un rebord inférieur (**12**) aménagés chacun pour présenter au moins une arête de rétention (**13**) s'étendant sensiblement dans le plan sagittal, autorisant une introduction par une voie postérieure,
**caractérisé en ce que** ladite paroi sagittale externe de l'implant présente :
- une face externe (**14**) aménagée pour présenter une conformation (**15**) d'aide à la pénétration permettant un décalage latéral de la cage jusqu'à une position de blocage dans l'espace discal, obtenue par l'arête de rétention (**13**) empêchant le recul latéral de la cage.

2. Implant selon la revendication 1, **caractérisé en ce que** la cage (**1**) présente une symétrie par rapport à un plan longitudinal (**L**) médian passant par les parois sagittales, pour permettre par son retournement, un positionnement symétrique avec une autre cage analogue (**1'**).

3. Implant selon la revendication 1 ou 2, **caractérisé en ce que** la cage (**1**) comporte au moins une paroi transversale intermédiaire (**6**) reliée aux parois sagittales (**2, 3**) et s'étendant à distance des parois antérieure (**5**) et postérieure (**4**) pour délimiter avec chacune d'entre elles, un volume de remplissage osseux (**7**).

4. Implant selon l'une des revendications 1 à 3, **caractérisé en ce que** la cage comporte une arête de rétention (**13**) s'étendant en saillie par rapport aux plans d'extension extrêmes inférieur (**P**_{**2**}) et supérieur (**P**_{**1**}) des parois transversales.

5. Implant selon l'une des revendications 1 à 3, **caractérisé en ce que** la cage (**1**) comporte une arête de rétention (**13**) obtenue par un biseautage réalisé dans les parties de raccordement (**17**) des parois transversales (**4, 5, 6**) avec la paroi sagittale dite externe (**3**).

6. Implant selon l'une des revendications 1 à 4, **caractérisé en ce que** chaque arête de rétention (**13**) réalisée sur la paroi sagittale externe (**3**) est prolongée transversalement vers l'extérieur, par une conformation d'aide à la pénétration (**15**) constituée par un chanfrein de sorte que ladite paroi sagittale (**3**) présente un profil tronconique.

7. Implant selon la revendication 6, **caractérisé en ce que** chaque arête de rétention (**13**) s'étend selon le rebord de la paroi sagittale externe, de façon continue ou en plusieurs tronçons entre la paroi antérieure (**5**) et la paroi postérieure (**4**).

8. Implant selon la revendication 7, **caractérisé en ce que** chaque arête de rétention (**13**) présente dans le plan sagittal, un profil rectiligne ou convexe.

9. Implant selon l'une des revendications 1 à 5, **caractérisé en ce que** chaque arête de rétention (**13**) est taillée localement, sensiblement perpendiculairement au plan sagittal (**S**), pour délimiter au moins un redent (**20**) s'opposant au recul de la cage dans le plan sagittal.

10. Implant selon la revendication 1 ou 4, **caractérisé en ce qu'**au moins l'une des parois transversales (**5, 6**) présente un rebord supérieur et un rebord inférieur, aménagés chacun pour présenter au moins une dent de rétention (**22**) empêchant le recul de la cage dans le plan sagittal.

11. Implant selon la revendication 10, **caractérisé en ce que** les parois transversales (**4, 5, 6**) présentent dans le plan sagittal (**S**) des hauteurs déterminées différentes définissant entre elles, une pente adaptée à la lordose physiologique de l'espace discale.

12. Implant selon la revendication 1 ou 4, **caractérisé en ce que** les parois sagittales (**2, 3**) sont pourvues de trous de passage (**10'**) débouchant à l'intérieur des volumes (**7**) de remplissage osseux pour favoriser la fusion osseuse.

## Patentansprüche

1. Zwischenkörperliches Lendenwirbelimplantat, das dazu bestimmt ist, in den zwischen zwei aneinander grenzenden Wirbeln definierten Wirbelscheibenraum eingesetzt zu werden, um den Zwischenwirbelraum anatomisch wiederherzustellen, wobei das Implantat in Form eines Gehäuses (1) von allgemeiner parallelepipedischer Form vorhanden ist, umfassend zwei Sagittalwände (2, 3), die miteinander durch mindestens eine quer verlaufende vordere Wand (5) und eine quer verlaufende hintere Wand (4) verbunden sind, wobei die Wände zwischen sich ein offenes Volumen (7) für eine Knochenauffüllung begrenzen,
wobei eine der Sagittalwände, äußere Wand (3) des Implantats genannt, aufweist:
- einen oberen Rand (11) und einen unteren Rand (12), die jeweils derart vorgesehen sind, dass sie mindestens eine Haltekante (13) aufweisen, die sich im Wesentlichen in der Sagittalebene erstreckt und eine Einführung von hinten gestattet,
wobei diese äußere Sagittalwand des Implantats aufweist:
- eine Außenfläche (14), die derart vorgesehen ist, dass sie eine Ausbildung (15) zur Unterstützung des Eindringens aufweist, die eine seitliche Versetzung des Gehäuses bis in eine Feststellposition in dem Wirbelscheibenraum ermöglicht, die durch die Haltekante (13), die das seitliche Abgleiten des Gehäuses verhindert, erhalten wird.

2. Implantat nach Anspruch 1, **dadurch gekennzeichnet, dass** das Gehäuse (1) eine Symmetrie in Bezug auf eine Längsmittelebene (L), die durch die Sagittalwände hindurch verläuft, aufweist, um, indem es umgedreht wird, eine zu einem weiteren vergleichbaren Gehäuse (1') symmetrische Positionierung zu ermöglichen.

3. Implantat nach Anspruch 1 oder 2, **dadurch gekennzeichnet, dass** das Gehäuse (1) mindestens eine quer verlaufende Zwischenwand (6) umfasst, die mit den Sagittalwänden (2, 3) verbunden ist und sich in einem Abstand zu der vorderen (5) und hinteren Wand (4) erstreckt, um mit jeder von ihnen ein Knochenbefüllungsvolumen (7) zu begrenzen.

4. Implantat nach einem der Ansprüche 1 bis 3, **dadurch gekennzeichnet, dass** das Gehäuse eine Haltekante (13) umfasst, die sich vorspringend in Bezug auf die äußerste untere (P₁) und obere (P₂) Ausdehnungsebene der quer verlaufenden Wände erstreckt.

5. Implantat nach einem der Ansprüche 1 bis 3, **dadurch gekennzeichnet, dass** das Gehäuse (1) eine Haltekante (13) umfasst, die durch eine Abschrägung erhalten wird, die in den Anschlussteilen (17) der quer verlaufenden Wände (4, 5, 6) mit der sogenannten äußeren Sagittalwand (3) hergestellt ist.

6. Implantat nach einem der Ansprüche 1 bis 4, **dadurch gekennzeichnet, dass** jede Haltekante (13), die auf der äußeren Sagittalwand (3) hergestellt ist, quer nach außen durch eine Ausbildung (15) zur Unterstützung des Eindringens verlängert ist, die durch eine Abfasung gebildet ist, so dass die Sagittalwand (3) ein kegelstumpfartiges Profil aufweist.

7. Implantat nach Anspruch 6, **dadurch gekennzeichnet, dass** sich jede Haltekante (13) entlang des Randes der äußeren Sagittalwand kontinuierlich oder in mehreren Abschnitten zwischen der vorderen Wand (5) und der hinteren Wand (4) erstreckt.

8. Implantat nach Anspruch 7, **dadurch gekennzeichnet, dass** jede Haltekante (13) in der Sagittalebene ein geradliniges oder konvexes Profil aufweist.

9. Implantat nach einem der Ansprüche 1 bis 5, **dadurch gekennzeichnet, dass** jede Haltekante (13) lokal im Wesentlichen senkrecht auf die Sagittalebene (S) eingeschnitten ist, um zumindest einen Absatz (20) zu begrenzen, der sich dem Abgleiten des Gehäuses in der Sagittalebene entgegen stellt.

10. Implantat nach Anspruch 1 oder 4, **dadurch gekennzeichnet, dass** mindestens eine der quer verlaufenden Wände (5, 6) einen oberen Rand und einen unteren Rand aufweist, die jeweils derart vorgesehen sind, dass sie mindestens einen Haltezahn (22) aufweisen, der das Abgleiten des Gehäuses in der Sagittalebene verhindert.

11. Implantat nach Anspruch 10, **dadurch gekennzeichnet, dass** die quer verlaufenden Wände (4, 5, 6) in der Sagittalebene (S) unterschiedliche bestimmte Höhen aufweisen, die zwischen sich ein Gefälle definieren, das an die physiologische Verkrümmung des Wirbelscheibenraumes angepasst ist.

12. Implantat nach Anspruch 1 oder 4, **dadurch gekennzeichnet, dass** die Sagittalwände (2, 3) mit Durchgangslöchern (10') versehen sind, die im Inneren der Knochenbefüllungsvolumen (7) münden, um die Knochenfusion zu begünstigen.

## Claims

1. Interbody vertebral implant for insertion in the disc space between two adjacent vertebrae with a view to restoring the anatomy of the intervertebral space, the implant being in the form of a cage (1) of general parallelepiped shape including two sagittal walls (2,3) joined together by at least one anterior transverse wall (5) and a posterior transverse wall (4), the walls delimiting between them an open volume (7) for bone filling, one of the sagittal walls, so-called external sagittal (3) wall of the implant having:
- an upper rim (11) and a lower rim (12) each arranged to have at least one retaining edge (13) extending substantially along the sagittal plane allowing insertion via a posterior approach,
**characterized in that** said sagittal external wall of the implant has:
- an outer face (14) arranged to have a conformation (15) to assist insertion allowing lateral offset of the cage as far as a locking position within the disc space, obtained by the retaining edge (13) preventing lateral recoil of the cage.

2. Implant as in claim 1, **characterized in that** cage (1) is symmetrical relative to a median longitudinal plane (L) passing through the sagittal walls so that, when reversed, it enables symmetrical positioning with another similar cage (1').

3. Implant as in claim 1 or 2, **characterized in that** the cage (1) comprises at least one intermediate transverse wall (6) joined to the sagittal walls (2, 3) and extending away from the anterior wall (5) and posterior wall (4) to delimit with each of the latter a bone filling volume (7).

4. Implant as in any of claims 1 to 3, **characterized in that** the cage comprises a retaining edge (13) that is raised relative to the lower (P₂) and upper (P₁) end planes of extension of the transverse walls.

5. Implant as in any of claims 1 to 3, **characterized in that** the cage (1) comprises a retaining edge (13) obtained by bevelling connection parts (17) of the transverse walls (4, 5, 6) with the so-called external sagittal wall.

6. Implant as in any of claims 1 to 4, **characterized in that** each retaining edge (13) made on the external sagittal wall (3) is extended transversely towards the outside by a conformation to assist insertion (15) consisting of a chamfer so that said sagittal wall (3) has a truncated cone profile.

7. Implant as in claim 6, **characterized in that** each retaining edge (13) extends along the rim of the external sagittal wall in continuous manner or in several segments between the anterior wall (5) and the posterior wall (4).

8. Implant as in claim 7, **characterized in that** each retaining edge (13) has a rectilinear or convex profile in the sagittal plane.

9. Implant as in any of claims 1 to 5, **characterized in that** each retaining edge (13) is locally cut substantially perpendicular to the sagittal plane (S) to delimit at least one retaining tooth (20) opposing recoil of the cage in the sagittal plane.

10. Implant as in claim 1 or 4, **characterized in that** at least one of the transverse walls (5, 6) has an upper rim and a lower rim each arranged to have at least one retaining tooth (22) preventing recoil of the cage in the sagittal plane.

11. Implant as in claim 10, **characterized in that** the transverse walls (4, 5, 6) in the sagittal plane (S) have different determined heights defining between them a slope adapted to the physiological lordosis of the disc space.

12. Implant as in claim 1 or 4, **characterized in that** the sagittal walls (2, 3) are provided with passage holes (10') opening into the inside of bone filling volumes (7) to promote bone fusion.
